Europäisches Patentamt

European Patent Office

Office européen des brevets

(11) Publication number: **0 278 653**
**A2**

## (12) EUROPEAN PATENT APPLICATION

(21) Application number: 88300840.1

(22) Date of filing: 02.02.88

(51) Int. Cl.⁴: **A61L 9/16** , A61L 9/12

(30) Priority: 03.02.87 IL 81466

(43) Date of publication of application:
17.08.88 Bulletin 88/33

(84) Designated Contracting States:
DE ES FR GB IT NL SE

(71) Applicant: LOG PLASTICS PRODUCTS, LTD.

Kibutz Ashdod Yaakov(IL)

(72) Inventor: Gross, Joseph
Moshav Mazor 205
73 160(IL)
Inventor: Tilman, Menahem
34 Ha Shaldag Street
Hofit(IL)
Inventor: Rosenberg, Etan
40 Ha Maagal Street
Rimon Kiryat Ono(IL)
Inventor: Lowenstein, David
41 Harav Kuk Street
Netanya(IL)

(74) Representative: Thomson, Paul Anthony et al
Potts, Kerr & Co. 15, Hamilton Square
Birkenhead Merseyside L41 6BR(GB)

(54) Air purifying apparatus.

(57) Air purifying apparatus comprising a base (12); a fan (14) supported by said base (12); and an air filtration element (40) supported by said base (12); and arranged to filter air supplied by said fan (14); and a lighting element (18,30).

## AIR PURIFYING APPARATUS

The present invention relates to air purifying systems that may be used to dispel foul odours in enclosed areas generally and in toilets in particular.

There are available on the market a wide range of devices for dispelling foul odours such as those that may be encountered in toilets. This range includes air-soluble fragrant blocks, air freshening sprays, and electric extractor fans which are often arranged to be activated together with a toilet light.

However, extractor fans need to have an outlet to a fresh air vent, and must be installed on an interior wall of the toilet. Additionally, the fan has to be connected to an electricity supply, and it may further be desired to wire the fan into a light switch such that the fan is activated together therewith as described above.

Furthermore, although an electric extractor fan is automatically operable and is generally considered to be efficient in removing foul odours from a toilet, it does not provide the additional fragrance that it may be desired to release into the toilet atmosphere. If it is desired to release a fragrant scent into the toilet atmosphere, it must be done by providing an air soluble fragrant block or an aerosol spray.

It is an object of the present invention to provide automatic air purifying apparatus that may be simply installed in a conventional light socket and that removes foul odours from an atmosphere while releasing a fragrance into the atmosphere.

There is thus provided, in accordance with an embodiment of the invention, air purifying apparatus comprising a base, a fan supported by the base, and an air filtration element supported by the base arranged to filter air supplied to it by the fan.

In accordance with an alternative embodiment of the invention, there is provided air purifying apparatus comprising a base, a fan supported by the base, an air filtration element supported by the base arranged to filter air supplied to it by the fan, and a lighting element.

In accordance with a preferred embodiment of the invention, there is also provided a fragrance-emitting element arranged downstream of the air filtration element for providing a fragrance to filtered air.

Additionally in accordance with a preferred embodiment of the invention, the air purifying apparatus additionally defines inflow and outflow vents.

Further in accordance with a preferred embodiment of the invention, the air filtration element and the fragrance-emitting element are provided upstream of the outflow vents.

Additionally in accordance with a preferred embodiment of the invention, the electric fan is configured to draw air inwards through the inflow vents and to force air outward along a flow path extending through the air filtration strip, the fragrance-emitting element and the outflow vents.

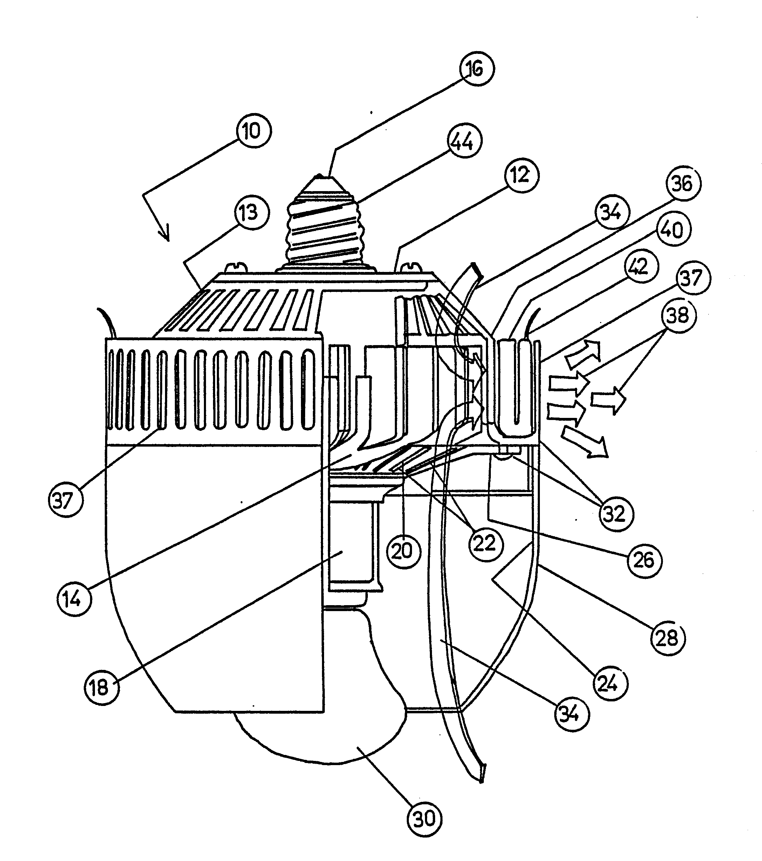

The present invention will be further illustrated, by way of example, with reference to the accompanying drawings, in which the single Figure is a partially cut-away illustration of an air-purifying apparatus according to a preferred embodiment of the present invention.

As illustrated, an air purifying apparatus according to a preferred embodiment of the invention is generally indicated by reference numeral 10. Air purifying apparatus 10 is shown to comprise a base element 12, from which is centrally suspended an electric fan 14, and to which there is radially attached a vented element 13.

Electric fan 14 is selectably rotatable about an axis 16 and a conventional light bulb holder 18 is mounted coaxially therewith. Provided on a lower face of electric fan 14 is a fan cover 20, in which there are a plurality of radially disposed inflow vents 22, the function of which will be described below.

Removable cover 24, typically supported by bracket element 26, comprises a lower portion 28, that acts as a shade for a light bulb 30, mounted in light bulb holder 18. Cover 24 further comprises an upper, vented conduit 32 radially aligned with respect to electric fan 14.

Electric fan 14 is configured to draw air inwards, through inflow vents 22 and through vented element 13, as indicated by arrows 34, and expel air through a plurality of inner and outer vents, referenced 36 and 37 respectively, radially disposed about vented conduit 32, as shown by arrows 38.

Retained within vented conduit 32 is a disposable air filtration strip 40 and a disposable fragrant strip 42. As air is expelled outwards through vents 36, any impurities in the air, typically resulting from foul odours as encountered in a toilet, are filtered off by filtration strip 40 which typically comprises activated charcoal.

As the air is further forced through fragrant strip 42, which typically comprises a strip of material impregnated with air-soluble fragrant nodules according to a desired fragrance, it adopts a fragrant odour and is expelled through vents 37 into the atmosphere. It should be noted that in a preferred embodiment of the invention filtration strip 40 and fragrant strip 42 are combined, in order that a user has only a single strip to replace once the filtering and fragrance-giving properties thereof begin to wear off.

According to a preferred embodiment of the invention, air purifying apparatus 10 may be installed in place of a conventional light bulb by means of a conventional light fitting 44, and thus may be installed as simply as a light bulb. When a person is occupying a room in which air purifying apparatus 10 has been installed, for example, a toilet, as soon as the toilet light is switched on air purifying apparatus 10 is operated, and similarly air purifying apparatus 10 ceases to operate once the light is switched off.

## Claims

1. Air purifying apparatus (10) comprising:
a base (12);
a fan (14) supported by said base, and
an air filtration element (40) supported by said base and arranged to filter air supplied by said fan (14).

2. Air purifying apparatus comprising:
a base (12);
a fan (14) supported by said base;
an air filtration element (40) supported by said base and arranged to filter air supplied by said fan (14); and
a lighting element (18,30).

3. Air purifying apparatus as claimed in claim 1 or 2, characterised in that said air filtration element comprises a strip element (40) arranged about said fan.

4. Air purifying apparatus as claimed in any preceding claim, characterised in that it also comprises a fragrance-emitting element (42) for providing a fragrance to filtered air.

5. Air purifying apparatus as claimed in claim 4, characterised in that said fragrance-emitting element (42) comprises a strip element disposed downstream of said air filtration element (40).

6. Air purifying apparatus as claimed in any preceding claim, characterised in that said air purifying apparatus additionally comprises a fan cover (20) having inflow vents (22).

7. Air purifying apparatus as claimed in claim 6, characterised in that said air purifying apparatus further comprises a removable housing (24) having outflow vents (37).

8. Air purifying apparatus as claimed in claim 7, characterised in that said air filtration strip element (40) and said fragrance-emitting strip element (42) are disposed upstream of said outflow vents (37).

9. Air purifying apparatus as claimed in claim 8, characterised in that said electric fan (14) is configured to draw air inwards through said inflow vents (22) and to force air outwards along a flow path extending through said air filtration strip (40), said fragrance-emitting strip (42) and said outflow vents (37).

10. Air purifying apparatus as claimed in any preceding claim, characterised in that said air filtration element (40) and said fragrance-emitting element (42) are disposable.

11. Air purifying apparatus as claimed in any preceding claim, characterised in that said air filtration element comprises activated charcoal.